# EUROPEAN PATENT SPECIFICATION

(11) **EP 1 239 791 B1**
(45) Date of publication and mention of the grant of the patent: **02.08.2006**
(21) Application number: 00989000.5
(22) Date of filing: 18.12.2000
(51) Int. Cl.: A61C 8/00, A61C 19/06

(54) **DENTAL PROSTHESIS WITH MEANS FOR THE RELEASE OF ACTIVE SUBSTANCES**
ZAHNPROTHESE MIT WIRKSTOFFABGABEVORRICHTUNG
PROTHESE DENTAIRE EQUIPEE DE MOYENS SERVANT A LIBERER DES SUBSTANCES ACTIVES

(30) Priority: 21.12.1999 IT FI990258
(43) Date of publication of application: 18.09.2002
(73) Proprietor: CONSIGLIO NAZIONALE DELLE RICERCHE, 00185 Roma (IT)
(72) Inventor: CHIARELLI, Piero, I-56017 S. Giuliano Terme (IT)
(74) Representative: Mannucci, Michele
(86) International application number: PCT/IT2000/000527
(87) International publication number: WO 2001/045585

(56) References cited:
- EP-A- 0 864 299
- WO-A-92/17127
- WO-A-97/22308
- NL-A- 9 500 256
- US-A- 5 584 688

## Description

### Technical field

The present invention relates to a prosthesis for dental implants that has the purpose of improving the outcome of an implantation procedure or of preventing the development of various kinds of complications following the implantation of a prosthetic structure.

### State of the art

Failure of a dental implant can be caused by imperfect stabilization at the time of intervention, but also by phenomena of bone resorption that appear some time after implantation. Loss of alveolar bone tissue, which acts as a support for the dental implants, can occur for physiological reasons connected with the patient's advanced age, or through necrosis following excessive compressive loading, which may be caused by inflammation and infections that originate on the surface of the gums and then advance, during their subsequent development, deeper and deeper towards the bone. The latter case can also occur even when surgical intervention has been executed correctly and in sterile conditions for implantation, since gum infections can arise in the post-operative period.

In both situations, the end result is an increase of the processes of resorption of the bone matrix, which oppose the activity of new bone formation around the prosthesis. In this case the result is loss both of extracellular matrix and of mineral component, as well as decrease in density and strength of the bone tissue.

Another possible cause of failure of a dental implant is the large difference in elastic modulus between bone (elastic modulus 17.4 GPa) and the titanium of which the prosthesis is made (the elastic modulus of which is approx. 105 GPa, compared with 19.8 GPa of the tooth). This important difference in elasticity between prosthesis and bone has an adverse effect on the latter, when it is subjected to stresses, producing microtraumas and micronecroses that tend to sclerotize the bone, weakening its mechanical structure, ultimately leading to mobility of the implant.

For the problems described above to be tackled effectively, it is important for the implantation zone to be protected constantly against the development of infections following the intervention, for stimulation of bone regrowth and its integration with the prosthesis as quickly as possible.

WO-A-9722308 discloses a prosthetic structure for dental implants according to the preamble of claim 1, comprising a polymeric base material incorporating at least one active component, wherein said base material releases said active component in a controlled manner when said prosthetic structure has been implanted in a living organism.

A different prosthesis is disclosed in NL-A-9500256.

### Summary of the invention

The above mentioned problems are solved by a prosthesis according to claim 1. The dependent claims relate to advantageous further features of the invention. The base material incorporating the active component to be released is a polymeric material, for example a material with the characteristics of a hydrogel. The active component can in general be a drug and in particular a drug whose function is to prevent or cure any inflammations of the bone tissue and/or of the soft tissue, especially the gums. According to a particular embodiment, the active component can be an antibiotic.

According to another aspect of the invention, the active component can be a component that acts as a metabolic stimulator of bone growth. In this last case the component can be selected for example among the morphogenetic proteins, the biphosphonates, osteogenetic proteins and/or their combinations.

The hydrogel or other controlled-release base material containing the active component can be located in a "seating" made in the portion of the prosthetic structure intended to be implanted in the bone, for example in the apical zone. Alternatively or in combination, it is possible to provide a seating that is made in the zone of the prosthetic structure that comes into contact with the gingival zone. In the first case the active component will be, advantageously, a metabolic stimulator of bone regrowth and will facilitate the reconstruction of the bone tissue around the zone of the prosthetic structure in contact with the actual bone. In the second case the active component will preferably be a drug with antibiotic action or the like, for preventing or curing the development of inflammations at the bone tissue level. In the second case the prosthetic structure can be of the type comprising a removable healing plug and the base material with the appropriate active component can be located in a seating made in this healing plug and can be removed once the definitive prosthesis is fitted permanently.

According to another aspect of the present invention, a part at least of the internal portion of the prosthetic structure intended to be implanted in the bone has a coating consisting of a base material incorporating, for example, a factor that has the function of a metabolic stimulator of bone regrowth. The coating can be made advantageously with a base material possessing an elastic modulus between 0.1 and 25 GPa and preferably between 0.5 and 1.5 GPa. The coating consists advantageously of a bioabsorbable base material, so that once implantation has been effected, the base material constituting the coating of the portion of the prosthesis in contact with the bone is gradually resorbed, while around the prosthesis itself there is reconstruction of bone tissue.

According to a perfected, particularly advantageous embodiment of the invention, a coating formed from the base material surrounding, at least partially, the internal portion of the prosthetic structure is loaded with microspheres of non-resorbable material. In this way the bone tissue that regrows following resorption of the base material will have a spongy, i.e. cellular, structure, corresponding to the non-resorbable microspheres. The microspheres can consist of a hydrogel or some other suitable material. The active component can be contained in the non-bioabsorbable microspheres.

It is clear from the foregoing that the prosthetic structure according to the present invention permits the use of biocompatible hydrogels that permit continuous controlled release of suitable drugs such as antibiotics and/or metabolic stimulators respectively in the gingival zone and in the zone in which the prosthesis is in contact with the bone.

In this connection, knowledge and techniques are now well consolidated in the field of biocompatible and/or bioresorbable polymeric materials for the release and dosage of pharmacological substances in the human body, but the use of these products in dental prostheses for the purposes described above has not been proposed. A hydrogel suitable for use in the present invention is described in EP-A-0 058 497.

The dosages and the times for *in situ* release of the substances contained in the hydrogels can be controlled by suitably varying the porosity of said materials or the kinetics of resorption in the case of bioresorbable polymers.

A range of implantation devices can be assembled on the basis of the modular modifications according to the present invention, depending on the various requirements. The structural modifications that have been developed can be applied to a great many commercially-available prostheses, which have in general tackled, comprehensively and exhaustively, the problem of mechanical design and of purity of the material, but have not tackled the case of pharmacological interaction with the surrounding system with which they will interact.

### Brief description of the drawings

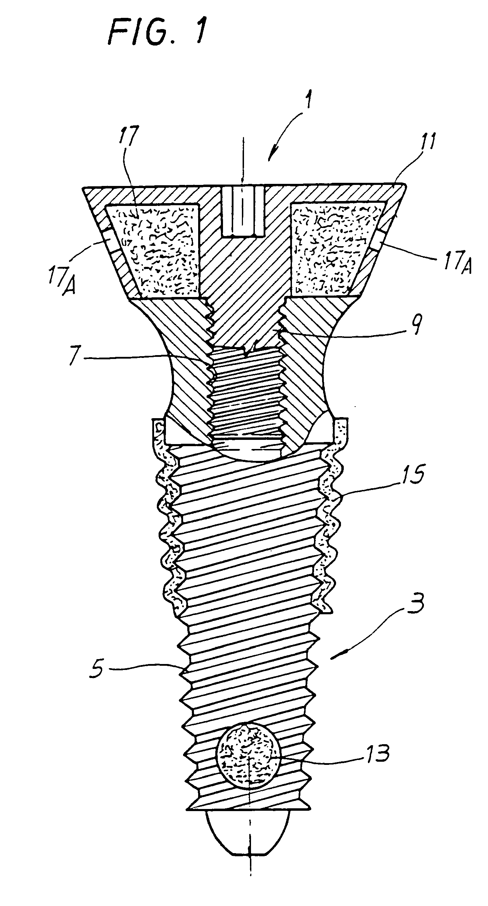

The invention will be better understood by following the description and the appended drawing, which shows a practical, non-limiting example of the said invention. In the drawing, the single diagram shows, in longitudinal section and partial view, a prosthetic structure according to the invention.

### Detailed description of the preferred embodiment of the invention

A prosthetic structure of the type with a healing plug is shown in the diagram and is designated 1. The prosthetic structure has a body 3 defining an apical portion and intended to be implanted in the bone. The body 3 has a threaded stem 5 and a threaded internal hole 7. A threaded stem 9 of a healing plug 11 engages with hole 7. The healing plug is screwed into body 3 of the prosthetic structure 1 and is held in this position for the necessary time prior to its replacement with the upper portion of the prosthesis that replaces the tooth.

According to a first aspect of the present invention, near the apical extremity of the body 3 of the prosthetic structure 1, a seating 13 is provided, in which a base material is inserted, for example a hydrogel, containing an active component, such as an antibiotic or a metabolic stimulator of bone regrowth. Bearing in mind that this prosthetic portion will be implanted in the bone tissue, it will be preferable to employ, as active component in this seating, a metabolic stimulator of regrowth rather than a drug possessing an antibiotic function.

According to another aspect of the present invention, around an intermediate zone of portion 3 of the prosthetic structure 1, a coating 15 is provided, consisting of a base material, which can for example be the same material inserted in seating 13, or a different material, but nevertheless having the function of continuous, controlled release of an active component which, also in this case, can be a stimulator of bone regrowth.

Microspheres of a non-bioabsorbable gel can be embedded in coating 15, made for example of a bioabsorbable gel. In this case the active component can be embedded in one or the other of the two gels and preferably in the gel constituting the microspheres.

When the prosthetic structure has been implanted, the gradual release of the regrowth factor from the coating 15 and/or from the seating 13 facilitates reconstruction of the bone tissue. The latter regrows around the body 3 of prosthetic structure 1 and comes to occupy the space progressively liberated by the bioabsorbable gel forming the coating 15. The possible presence of microspheres inside this coating leaves zones in which the bone tissue does not regrow, and hence assumes a porous structure. By suitably selecting the elastic modulus of the material constituting the microspheres, a structure is obtained with optimum elasticity for correct functioning of the prosthesis.

In essence the coating 15 has a function of shock absorber between the prosthetic structure and the bone and limits bone traumas when the prosthesis is submitted to stresses, for example during mastication. This coating makes it possible to solve the problems connected with absence of the periodontium, i.e. of the zone of the bone surrounding the tooth that is replaced in this case by the prosthetic structure. The periodontium has an elastic modulus of the order of 0.8 GPa and so is softer than true bone (elastic modulus 17 GPa) and tooth (19.8 GPa).

The coating 15 made with a suitable elastic modulus and with a thickness between for example 1 and 500 microns, makes it possible to eliminate the problems arising from the difference in elastic modulus between the titanium of the prosthetic structure and the actual bone. When, as mentioned above, the coating is made of a hydrogel or some other bioresorbable material loaded with non-resorbable microspheres, the residual microspheres permit reconstruction of the tissue of the spongy tissue that accordingly creates an interface between the prosthetic structure and the pure bone, with a shock-absorbing behavior very similar to that of the natural periodontium.

The coating 15 can also be made with a material with a suitable elastic modulus and thickness for constituting a long-lasting shock-absorbing layer. In this case it is preferably constituted of a non-bioresorbable material. If its only function is shock absorbing, it will not contain any active component to be released once the prosthesis is implanted. In this case the coating 15 can be used alone or in combination with a base material that releases an active component disposed at other points of the prosthesis, as described in this context.

According to a third aspect of the present invention, in the upper zone of the prosthetic structure 1 consisting in the present case of the healing plug 11, another seating 17 is provided for receiving a base material, for example a hydrogel, loaded with an active component that is gradually released. In the example illustrated, the seating 17 has an annular development and has holes 17A by which the interior of the seating 17 communicates with the surrounding gingival tissues once the prosthetic structure has been implanted. The active component loading the base material contained in seating 17 is released in a gradual and controlled manner through the holes 17A. This active component can consist of an anti-inflammatory drug, an antibiotic, and/or of stimulators of tissue regrowth. The holes 17A are of a suitable size, and preferably larger than 0.3 mm and are arranged laterally all the way along the annular development of the healing plug 11.

The prosthetic structure shown in the drawing has both the seating 13 in the zone intended to be implanted in the bone, and the seating 17 in the outer zone of the prosthesis, as well as the coating 15. It should, moreover, be understood that these three aspects can also be used individually or in combination two by two.

For each of the three applications it is possible to use hydrogels of various kinds as well as active components of various types according to the specific application.

In the seating 17 of the healing plug 11 it is possible to insert a hydrogel based on polyvinyl alcohol (PVA) incorporating an antibiotic, or equivalent active component, for example metronidazole.

In the seating 13 made in the apical zone of the body 3 of the prosthesis it is moreover possible to use a hydrogel based on polyvinyl alcohol but advantageously replacing the metronidazole with a bone regrowth factor such as the "bone morfonegenetic proteins" (BMP), i.e. bone morphogenetic proteins, or other active components possessing similar functions such as bisphosphonates, osteogenetic proteins or other components possessing bone tissue stimulation function.

For the seating 15 made around a zone of the body 3 of the prosthesis it is possible to use polylactic acid in conjunction with amelogenin for stimulation of regrowth of the periodontal ligament during spontaneous resorption of the polylactic acid. Alternatively, as mentioned above, the polylactic acid can be loaded with microspheres of non-resorbable hydrogels containing stimulators of bone regrowth. Replacement of the polylactic acid in the spaces around the microspheres with bone tissue tends to create an elastically yielding alveolar bone structure and we thus obtain a lowering of the level of total load on the bone tissue surrounding the prosthesis during the torsional stresses exerted for example during mastication. In both cases the objective is to create a cushioned zone between the prosthetic structure and the bone with shock absorbing function.

It is to be understood that the drawing only shows one example given purely as practical demonstration of the invention, it being possible for this invention to vary in its forms and arrangements but without leaving the scope of the concept taught by the said invention.

## Claims

1. A prosthetic structure for dental implants comprising a polymeric base material incorporating at least one active component, wherein said base material releases said active component in a controlled manner when said prosthetic structure has been implanted in a living organism, **characterized in that** said base material is a polymeric material with the characteristics of a hydrogel.

2. Prosthetic structure as in claim 1, including a seating for said base material

3. Prosthetic structure as in claim 1 or 2. wherein said at least one active component is an antibiotic.

4. Prosthetic structure as in Claim 3, wherein said active component is metronidazole.

5. Prosthetic structure as in one or more of the preceding claims, wherein said active component is a metabolic stimulator of bone regrowth.

6. Prosthetic structure as in Claim 5, wherein said active component is selected from the group comprising: morphogenetic proteins, bisphosphonates, osteogenetic proteins, amelogenin, or their combinations.

7. Prosthetic structure as in one or more of the preceding claims, comprising an internal portion intended to be implanted in the bone, said seating being arranged in said internal portion and being provided with an opening to the outside of the prosthesis.

8. Prosthetic structure as in one or more of the preceding claims, comprising an outer portion intended to come into contact with the gingival zone, said seating being arranged in said outer portion and being provided with an opening to the outside of the prosthesis.

9. Prosthetic structure as in Claim 8, wherein said outer portion includes a removable healing plug.

10. Prosthetic structure as in one or more of the preceding claims, including an inner portion intended to be implanted in the bone, there being applied on at least one zone of said inner portion, a coating consisting of said base material.

11. Prosthetic structure as in Claim 10, wherein said coating consists of a material possessing an elastic modulus between 0.1 and 25 GPa and preferably between 0.5 and 1.5 GPa.

12. Prosthetic structure as in Claim 10 or 11, wherein said coating consists of a bioresorbable material.

13. Prosthetic structure as in Claim 12, wherein said coating is loaded with microspheres of non-bioresorbable material.

14. Prosthetic structure as in Claim 13, wherein said microspheres consist of a hydrogel.

15. Prosthetic structure as in Claim 13 or 14, wherein the active component is contained in said microspheres.

## Patentansprüche

1. Prothesenstruktur für Dentalimplantate, umfassend ein polymeres Basismaterial, das mindestens eine aktive Komponente enthält, wobei das Basismaterial die aktive Komponente gesteuert freigibt, wenn die Prothesenstruktur in einen lebenden Organismus implantiert worden ist, ***dadurch gekennzeichnet,* dass** das Basismaterial ein Polymermaterial mit den Eigenschaften eines Hydrogels ist.

2. Prothesenstruktur nach Anspruch 1, enthaltend einen Sitz für das Basismaterial.

3. Prothesenstruktur nach Anspruch 1 oder 2, wobei die mindestens eine aktive Komponente ein Antibiotikum ist.

4. Prothesenstruktur nach Anspruch 3, bei der die aktive Komponente Metronidazol ist.

5. Prothesenstruktur nach einem oder mehreren der vorangehenden Ansprüche, bei der die aktive Komponente ein metabolischer Stimulator für Knochen-Nachwachsen ist.

6. Prothesenstruktur nach Anspruch 5, bei der die aktive Komponente ausgewählt ist aus der Gruppe, enthaltend morphogenetische Proteine, Biphosphonate, osteogenetiscue Proteine, Amelogenin, oder Kombinationen davon.

7. Prothesenstruktur nach einem oder mehreren der vorangehenden Ansprüche, umfassend einen inneren Abschnitt, der zum Implantieren in dem Knochen bestimmt ist, wobei der Sitz in dem inneren Abschnitt angeordnet und mit einer Öffnung zur Außenseite der Prothese versehen ist.

8. Prothesenstruktur nach einem oder mehreren der vorangehenden Ansprüche, umfassend einen äußeren Abschnitt, der für den Kontakt mit dem Zahnfleischbereich bestimmt ist, wobei der Sitz in dem äußeren Bereich angeordnet und mit einer Öffnung zur Außenseite der Prothese versehen ist.

9. Prothesenstruktur nach Anspruch 8, bei der der äußere Abschnitt einen entfernbaren Einheilstopfen enthält.

10. Prothesenstruktur nach einem oder mehreren der vorangegangenen Ansprüche, mit einem inneren Abschnitt, der zu Implantieren in den Knochen bestimmt ist, wobei an mindestens einer Zone des inneren Abschnitts eine Beschichtung angebracht ist, die aus dem Basismaterial besteht.

11. Prothesenstruktur nach Anspruch 10, bei der die Beschichtung aus einem Material mit einem Elastizitätsmodul zwischen 0,1 und 25 GPa, vorzugsweise zwischen 0,5 und 1,5 GPa, besteht.

12. Prothesenstruktur nach Anspruch 10 der 11, bei der die Beschichtung aus einem biologisch resorbierbaren Material besteht.

13. Prothesenstruktur nach Anspruch 12, bei der der Beschichtung Mikrosphären aus nicht biologisch resorbierbarem Material beigegeben sind.

14. Prothesenstruktur nach Anspruch 13, bei der die Mikrosphären aus einem Hydrogel bestehen.

15. Prothesenstruktur nach Anspruch 13 oder 14, bei der die aktive Komponente in den Mikrosphären enthalten ist.

## Revendications

1. Structure prosthétique pour des implants dentaires comprenant un matériau de base polymère incorporant au moins un composant actif, dans lequel ledit matériau de base libère ledit composant actif de manière contrôlée lorsque ladite structure prosthétique a été implantée dans un organisme vivant, **caractérisée en ce que** ledit matériau de base est un matériau polymère ayant les caractéristiques d'un hydrogel.

2. Structure prosthétique selon la revendication 1, incluant une assise pour ledit matériau de base.

3. Structure prosthétique selon la revendication 1 ou la revendication 2, ledit au moins un composant actif étant un antibiotique.

4. Structure prosthétique selon la revendication 3, ledit composant actif étant le métronidazole.

5. Structure prosthétique selon une ou plusieurs des revendications précédentes, ledit composant actif étant un stimulateur métabolique de la croissance nouvelle de l'os.

6. Structure prosthétique selon la revendication 5, ledit composant actif étant choisi dans le groupe comprenant : des protéines morphogénétiques, des bisphosphonates, des protéines ostéogéniques, l'amélogénine ou leurs associations.

7. Structure prosthétique selon une ou plusieurs des revendications précédentes, comprenant une partie interne destinée à être implantée dans l'os, ladite assise étant arrangée dans ladite partie interne et étant dotée d'une ouverture vers l'extérieur de la prothèse.

8. Structure prosthétique selon une ou plusieurs des revendications précédentes, comprenant une partie externe destinée à entrer en contact avec la zone gingivale, ladite assise étant arrangée dans ladite partie externe et étant dotée d'une ouverture vers l'extérieur de la prothèse.

9. Structure prosthétique selon la revendication 8, ladite partie externe incluant un bouchon de cicatrisation amovible.

10. Structure prosthétique selon une ou plusieurs des revendications précédentes, incluant une partie interne destinée à être implantée dans l'os, un revêtement consistant en ledit matériau de base étant appliqué sur au moins une zone de ladite partie interne.

11. Structure prosthétique selon la revendication 10, ledit revêtement consistant en un matériau possédant un module élastique entre 0,1 et 25 GPa et de préférence entre 0,5 et 1,5 GPa.

12. Structure prosthétique selon la revendication 10 ou la revendication 11, ledit revêtement consistant en un matériau biorésorbable.

13. Structure prosthétique selon la revendication 12, ledit revêtement étant chargé de microsphères de matériau non biorésorbable.

14. Structure prosthétique selon la revendication 13, lesdites microsphères consistant en un hydrogel.

15. Structure prosthétique selon la revendication 13 ou la revendication 14, le composant actif étant contenu dans lesdites microsphères.
